# EUROPEAN PATENT APPLICATION

(11) **EP 1 193 249 A1**
(43) Date of publication of application: **03.04.2002**
(21) Application number: 00121566.4
(22) Date of filing: 02.10.2000
(51) Int. Cl.: C07C 275/16, C12P 13/00, C12P 13/10, C12N 1/20, A61K 31/17, A61K 31/197

(54) **Citrullimycines, a process for their production and their use as pharmaceuticals**

(71) Applicant: Aventis Pharma Deutschland GmbH, 65929 Frankfurt am Main (DE)
(72) Inventor: Hopmann,Cordula Dr., 60322 Frankfurt (DE); Kurz, Michael Dr., 65719 Hofheim (DE); Brönstrup, Mark Dr., 60316 Frankfurt (DE); Wink, Joachim Dr., 63322 Rödemark (DE)

(57) **Abstract**

The present invention relates to a compounds named Citrullimycines which are obtainable by cultivation of *Streptomyces* sp. ST 101396 (DSM 13309), and to their pharmaceutically acceptable salts. The present invention further relates to a process for the production of the Citrullimycines, to the microorganism *Streptomycetes sp.,ST* 101396 (DSM 13309), to the use of the Citrullimycines and their pharmaceutically acceptable salts as a pharmaceutical, in particular to their use as inhibitors of the neurotensin receptor, and to pharmaceutical compositions comprising Citrullimycines or a pharmaceutically acceptable salt thereof.

## Description

The present invention relates to novel active compounds (Citrullimycines) which are obtainable by cultivation of *Streptomycetes* sp., ST 101396 (DSM 13309), and to their pharmaceutically acceptable salts and derivatives. The present invention further relates to a process for the production of the Citrullimycines, to the *Streptomycetes* sp. ST 101396 (DSM 13309), to the use of the Citrullimycines as a pharmaceutical, in particular to their use as substances with an affinity for neurotensin receptors, and to Citrullimycine-containing pharmaceuticals.

Neurotensin is a brain and gastrointestinal 13-amino acid hormonal peptide, which is involved in the control of a broad variety of physiological activities as a central neurotransmitter or neuromodulator in both the central nervous system and in the periphery. It fulfills many functions through their interaction with specific receptors which have been characterized in several tissues and cell lines of peripheral and central organs. Studies with neurotensin have led to implications of their involvement in schizophrenia, Parkinson and Alzheimer diseases. Compounds with an affinity for the neurotensin receptors might therefore be useful as therapeutics of these diseases.

It has now been found that the microorganism strain *Streptomycetes sp.,* ST101396, DSM 13309, is able to form novel active substances which inhibit the human neurotensin receptor proteins expressed in Human Embryonic Kidney (HEK) cell membranes.

The present invention accordingly relates to the active substances (Citrullimycines) formed by the strain *Streptomycetes sp.,* DSM 13309, and to their physiologically tolerated salts, esters, ethers and other obvious chemical equivalents.

The present invention accordingly relates to compounds of the formula I 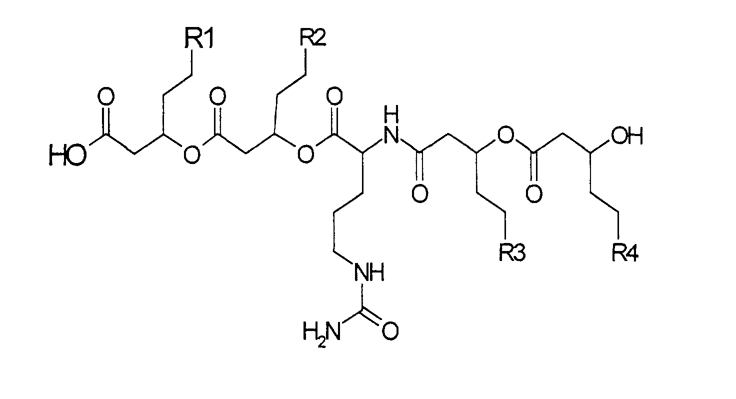 where
R₁, R₂, R₃ and R₄ are, independently of one another, alkyl residues with 1 to 6, preferably 2 to 5, carbon atoms;
and the physiologically tolerated salts thereof and derivatives such as esters, ethers and other obvious chemical equivalents, including all stereoisomeric forms and all tautomeric forms.

The alkyl residues in the compounds of the formula I can be straight-chain or branched.

Examples of alkyl residues are: methyl, ethyl, propyl, butyl, pentyl, hexyl, isopropyl, isobutyl, isopentyl, sec-butyl, tertbutyl and neopentyl.

The invention further relates to
1) a compound of the formula I above in which one of the residues R₁, R₂, R₃ or R₄ is a straight or branched propyl residue and the rest of the residues are straight or branched butyl residues ( =Citrullimycine A: molecular formula: C₄₁H₇₅N₃O₁₁, MW: 785) and the physiologically tolerated salts and derivatives thereof;
2) a compound of the formula I above in which either:
   (a) two of the residues R₁, R₂, R₃ or R₄ are straight or branched butyl residues and two of the residues are straight or branched propyl residues; or
   (b) R₁, R₂, R₃ and R₄ are one butyl, one pentyl , one ethyl and one propyl residue, in any order, the residues being either straight chain or branched; (= Citrullimycine B: molecular formula: C₄₀H₇₃N₃O₁₁, MW 771) and the physiologically tolerated salts and derivatives thereof; and
3) a compound of the formula I above in which either:
   (a) R₁, R₂, R₃ and R₄ are straight or branched butyl residues; or
   (b) two of the residues R₁, R₂, R₃ and R₄ are straight or branched butyl residues, one is a straight or branched propyl residue and one is a straight or branched pentyl residue;
      (= Citrullimycine C: molecular formula: C₄₂H₇₇N₃O₁₁, MW 799) and the physiologically tolerated salts and derivatives thereof.

It is, of course, understood that the compounds of formula I may exist in a variety of isomeric configurations, including structural as well as stereo-isomers. It is further understood that the present invention encompasses those compounds of formula I in each of their various structural and stereo isomeric configurations as individual isomers and as mixtures of isomers.

The Citrullimycines A to C according to the present invention are generally isolated as a mixture of isomers. In respect of Citrullimycine A, for example, an isolated sample may comprise a mixture of two or more of the following isomers:
Isomer 1: R₁, R₂ and R₄ = -CH(CH₃)CH₂CH₃, R₃ = -CH(CH₃)CH₃
Isomer 2: R₁, R₂ and R₃ = -CH(CH₃)CH₂CH₃, R₄ = -CH(CH₃)CH₃
Isomer 3: R₁, R₃ and R₄ = -CH(CH₃)CH₂CH₃, R₂ = -CH(CH₃)CH₃
Isomer 4: R₁ = -CH(CH₃)CH₃, R₂, R₃ and R₄ = -CH(CH₃)CH₂CH₃

The isomers of Citrullimycine A may exist in any ratio in the mixture isolated.

Citrullimycines A-C may be characterized by any one or more of their physicochemical and spectral properties, such as their mass spectrometry or ¹H NMR and ¹³C NMR spectroscopic data ( see Tables 1 and 2 below ).

The compounds of the formula I may be described as a sequence of four β-hydroxyacids with a citrulline molecule incorporated in the middle of the sequence. Citrullimycines A-C differ in the length of the alkylchain of the β-hydroxyacids. The compounds of the formula I are obtainable by cultivation of the microorganism *Streptomycetes sp.* ST 101396 (DSM 13309). The said microorganism has been deposited on the 14 February 2000 with the German Collection of Microorganisms and Cell Cultures (DSMZ - Deutsche Sammlung von Mikroorganismen und Zellkulturen GmbH), Braunschweig, Germany and has been given the accession number DSM 13309.

Thus, the present invention further provides a process for the production of said compounds of the formula I, which comprises cultivating the microorganism *Streptomycetes species* DSM 13309, its mutants and variants, under aerobic conditions in a nutrient medium containing one or more sources of carbon and one or more sources of nitrogen and, optionally, nutrient inorganic salts and/or trace elements, and then isolating and purifying the said compounds in a customary manner.

The nutrient medium preferably contains sources of carbon, nitrogen and nutrient inorganic salts. The carbon sources are, for example, starch, glucose, sucrose, dextrin, fructose, molasses, glycerol, lactose or galactose, preferably glucose. The sources of nitrogen are, for example, soyabean meal, peanut meal, yeast extract, beef extract, peptone, malt extract, corn steep liquor, gelatin or casamion acids, preferably soyabean meal. The nutrient inorganic salts are, for example sodium hydrogen phosphate, potassium hydrogen phosphate, ammonium hydrogen phosphate, sodium chloride, calcium chloride, calcium carbonate, potassium nitrate, ammonium sulphate or magnesium sulphate, preferably calcium carbonate and cobalt(II)chloride.

The cultivation of strain DSM 13309 may be carried out at temperatures between 20-35°C and pH between 5.0 and 8.0. Preferably strain DSM 13309 is cultivated at 27°C (±1°C) and pH 6.8-7.0.

The cultivation of strain DSM 13309 is preferably carried out for 48-240 hours when an optimal yield of the Citrullimycines of the invention is obtained. It is particularly preferred to carry out the cultivation by fermentation for 60-120 hours under submerged conditions for example in shake flasks as well as in laboratory fermenters. The progress of fermentation and formation of the Citrullimycines can be detected by High Pressure Liquid Chromatography (HPLC) or LC-MS and by measuring the bioactivity of the culture broth. In the resulting culture broth The Citrullimycines are present in the culture filtrate as well as in the mycelium. They can be isolated using known separation techniques. Thus, they can be recovered from the culture filtrate by extraction with a water immiscible solvent such as ethyl acetate, dichloromethane, chloroform or butanol at pH 3-8 or by hydrophobic interaction chromatography using polymeric resins such as "Diaion HP-20® " or "MCI® Gel CHP-20P" (Mtheirubishi Chemical Industries Limited, Japan), "Amberlite XAD® " (Rohm and Hass Industries U.S.A.), activated charcoal or ion exchange chromatography at pH 3-8. The preferred method is chromatography on MCI® Gel CHP-20P. The active material can also be recovered from mycelium by extraction with a water miscible solvent such as methanol, acetone, acetonitrile, *n*-propanol or *iso*-propanol or a water immiscible solvent such as ethyl acetate, dichloromethane, chloroform or butanol at pH 3-8 and the preferred method is the extraction with methanol. Concentration and lyophilization of the extracts gives the active crude material.

The Citrullimycines of the present invention may, for example, be recovered from the crude material as follows :

By fractionation using any of the following techniques: normal phase chromatography (using alumina or silica gel as stationary phase and eluents such as petroleum ether, ethyl acetate, methylene chloride, acetone, chloroform, methanol or combinations thereof and additions of amines such as NEt₃), reverse phase chromatography (using reverse phase silica gel like dimethyloctadecylsilylsilica gel, also called RP-18 or dimethyloctylsilyl silica gel also called RP-8 as stationary phase and eluents such as water, buffers viz. phosphate, acetate, citrate (pH 2-8) and organic solvents such as methanol, acetonitrile, acetone, tetrahydrofuran or combinations of these solvents), gel permeation chromatography using resins such as ®Sephadex LH-20 (Pharmacia Chemical Industries, Sweden), TSKgel ®Toyopearl HW (TosoHaas, Tosoh Corporation, Japan) in solvents such as methanol, chloroform, acetone, ethyl acetate or their combinations or ®Sephadex G-10 and G-25 in water; or by counter-current chromatography using a biphasic eluent system made up of two or more solvents such as water, methanol, ethanol, *iso*-propanol, *n*-propanol, tetrahydrofuran, acetone, acetonitrile, methylene chloride, chloroform, ethyl acetate, petroleum ether, benzene and toluene. These techniques may be used repeatedly or a combination of the different techniques may be used. The preferred method is chromatography over reverse phase silica gel (RP-18).

Obvious chemical equivalents ('derivatives') of the compounds according to the invention are compounds which show slight chemical difference, that is to say have the same effect or are converted under mild conditions into the compounds according to the invention. Said equivalents include, for example, esters, ethers, complexes or adducts of the or with the compounds according to the invention.

The compounds according to the present invention may be converted into pharmaceutically acceptable salts and derivatives, which are all covered by the present invention. The salts and derivatives can be prepared by standard procedures known to one skilled in the art.

Physiologically tolerated salts of the compounds of the formula I mean both the organic and the inorganic salts thereof as described in Remington's Pharmaceutical Sciences (17^{th} edition, page 1418 (1985)). Salts like sodium and potassium salts, for example, may be prepared by treating the compounds according to the invention with suitable sodium or potassium bases.

Esters of the free carboxylic acid may be prepared by methods given in the literature for example by treatment with an alcohol in the presence of a dehydrating agent like decyclohexylcarbodiimide (DCC) as described in Advanced Organic Synthesis, 4^{th} Edition, J. March, John Wiley & Sons., 1992, page 395.

The ester groups of the compounds according to the present invention may be reduced to ether groups as given in the literature, for example, by treatment with BF₃-etherat, LiAlH₄ LiBH₄ or NaBH₄ as described in Advanced Organic Synthesis, 4^{th} Edition, J. March, Wiley & Sons., 1992, page 1213. The amide group may be reduced in the same way by reaction with LiAlH₄.

The Citrullimycines according to the invention show inhibition in the neurotensin receptor binding assay of the human neurotensin receptor proteins expressed in the HEK cell membranes. In this assay, Citrullimycine A showed an IC₅₀ of 16 µM.

The compounds according to the present invention and their pharmaceutically acceptable salts and derivatives can be administered to animals, preferably to mammals, and in particular to humans as pharmaceuticals on their own, in mixtures with one another and in the form of pharmaceutical compositions which permit parenteral administration. Accordingly the present invention also relates to compounds of the formula I above and their pharmaceutically acceptable salts and derivatives for use as pharmaceuticals, in particular for use as a neurotensin antagonist with an affinity for the neurotensin receptor. The present invention further relates to pharmaceutical compositions which contain an effective amount of one or more of the target compounds and/or one or more pharmaceutically acceptable salts and/or derivatives thereof, together with a pharmaceutically acceptable carrier.

The compounds according to the invention can be administered orally, intramuscularly, intravenously or by other modes of administration. Pharmaceutical compositions which contain these compounds or a pharmaceutically acceptable salt or derivative thereof, optionally with other pharmaceutically active substances, can be prepared by mixing the active compounds with one or more pharmacologically tolerated auxiliaries and/or excipients. The mixture can then be converted into a suitable pharmaceutical form such as tablets, coated tablets, capsules, granules, powders, emulsions, suspensions or solutions.

Examples of auxiliaries and/or excipients which may be mentioned are fillers, emulsifiers, lubricants, masking flavours, colorants and buffer substances tragacanth, lactose, talc, agar-agar, polyglycols, ethanol and water. Suitable and preferred for parenteral administration are suspensions or solutions in water. It is also possible to administer the active substances as such, without vehicles or diluents, in a suitable form, for example, in capsules.

The invention also relates to a method for the production of a pharmaceutical, which comprises converting at least one of the compounds according to the invention with a pharmaceutically suitable and physiologically tolerated carrier and, where appropriate, further suitable active substances, additives or excipients into a suitable dosage form.

As is customary, the galenic formulation and the method of administration as well as the dosage range which are suitable in a specific case depend on the species to be treated and on the state of the respective condition or disease, and can be optimized using methods known in the art.

The following are illustrative examples of the present invention but not limitative of the scope thereof:

### EXAMPLE 1

### Maintenance of the producer strain Streptomycetes species ST 101396, DSM 13309

### Composition of maintenance medium

The producer strain DSM 13309 was maintained on the following medium :

| | |
|---|---|
| Malt extract | 10.0 g |
| Glucose | 4.0 g |
| Yeast extract | 4.0 g |
| Agar powder | 15.0 g |
| Demineralised water | 1 litre |
| pH | 7.0-7.5 |

After dissolving the ingredients thoroughly by heating, the resultant solution was distributed in test tubes and sterilized at 121°C for 20 mins. The test tubes were cooled and allowed to solidify in a slanting position. The agar slants were streaked with the growth of *Streptomycetes sp*.,ST 101 396, DSM 13309, by a wire loop and incubated at 28°C (±1°C) until a good growth was observed. The well grown cultures were stored in the refrigerator at +8°C.

### Preparation of glycerol working seed

| Composition of medium | |
|---|---|
| Yeast extract | 4 g |
| Soluble starch | 15 g |
| K₂HPO₄ | 1 g |
| MgSO₄ x 7 H₂O | 0.5 g |
| Demineralised water | 1 litre |
| PH | 7.0 |

The above medium was distributed in 100 ml amounts in 300 ml Erlenmeyer flasks and autoclaved at 121°C for 20 minutes. The flasks were cooled to room temperature and inoculated with the above mentioned agar slant. The incubation was carried out for five days on a rotary shaker at 180 rpm and 28°C. 1.5 ml of this culture were mixed with 1.5 ml glycerol (99 %) and stored at -20°C.

### EXAMPLE 2

### Fermentation of the strain Strepotomycetes sp., ST 101396, DSM 13309 in shake flasks

| Composition of seed medium: | |
|---|---|
| Glucose | 20 g |
| Soyabean meal | 10 g |
| CaCO3 | 0.02 g |
| CoCl₂ x 6 H₂O | 0.001 g |
| Demineralised water | 1 litre |
| pH | 6.8 - 7.0 |

The above medium was distributed in 100 ml amounts in 500 ml Erlenmeyer flasks and autoclaved for 20 mins. The flasks were cooled to room temperature and each flask was inoculated with a loopful of the above mentioned well grown culture of Example 1 and shaken on a rotary shaker for 72 hours at 240 rpm at 27°C (±1°C) to give seed culture.

| Composition of production medium: | |
|---|---|
| Glucose | 20 g |
| Soyabean meal | 10 g |
| CaCO₃ | 0.02 g |
| CoCl₂ x 6 H₂O | 0.001 g |
| Demineralised water | 1 litre |
| pH | 6.8 - 7.0 |

The production medium was distributed in 100 ml amounts in 500 ml Erlenmeyer flasks and autoclaved at 121°C for 20 min. The flasks were cooled to room temperature and inoculated with the above mentioned seed culture(2% v/v).The fermentation was carried out on a rotary shaker at 240 rpm and 27°C (±1°C) for 72 hours. The production of the inhibitors Citrullimycine A-C was determined by testing their bioactivity .

### EXAMPLE 3

### Isolation and purification of the Citrullimycines A-C

The culture broth (3 litres) was harvested and freeze dried. The lyophilization product was extracted with methanol (3 litres) and the active extracts were pooled and concentrated under reduced pressure and freeze dried to yield 25 g of crude material. This crude material was purified by preparative HPLC using the following conditions:

| 1.) Column: MCI® Gel CHP-20P ( 600 x 40 mm; Kronlab) | | | |
|---|---|---|---|
| Eluent: | A) H₂O B) MeOH | | |

| Gradient: | min | %A | %B |
|---|---|---|---|
| | 0 | 100 | 0 |
| | 17.5 | 100 | 0 |
| | 17.6 | 80 | 20 |
| | 40 | 80 | 20 |
| | 40.1 | 50 | 50 |
| | 55 | 50 | 50 |
| | 55.1 | 30 | 70 |
| | 67.5 | 30 | 70 |
| | 67.6 | 15 | 85 |
| | 72.5 | 15 | 85 |
| | 72.6 | 10 | 90 |
| | 77.5 | 10 | 90 |
| | 77.6 | 0 | 100 |
| Flow: 20 ml/min | | | |
| Detection : 358 nm | | | |

The active fractions eluted after 75 min. The pooled fractions were concentrated under reduced pressure and freeze dried.

Final purification was done by preparative HPLC using the following conditions:

| 1.) Column: Nucleosil 100-RP 18-AB (5 µ, 250 x 21 mm, Macherey & Nagel) | | | |
|---|---|---|---|
| Eluent: | A) H₂O B) CH₃CN | | |

| Gradient: | min | %A | %B |
|---|---|---|---|
| | 0 | 70 | 30 |
| | 22 | 70 | 30 |
| | 38 | 52 | 48 |
| | 44 | 52 | 48 |
| | 92 | 0 | 100 |
| | 110 | 0 | 100 |
| Flow Rate: 10 ml/min | | | |
| Detection: 360 nm | | | |

The active fractions were analyzed by LC-MS. The Citrullimycines A-C containing fractions eluted after 87 min (A), 97 min (B) and 98 min (C). The pooled fractions were concentrated under reduced pressure and freeze dried. The overall yield from 3 L culture broth was 1 mg of each substance.

The physico chemical and spectral properties of Citrullimycine A-C are given in Tables 1 and 2.

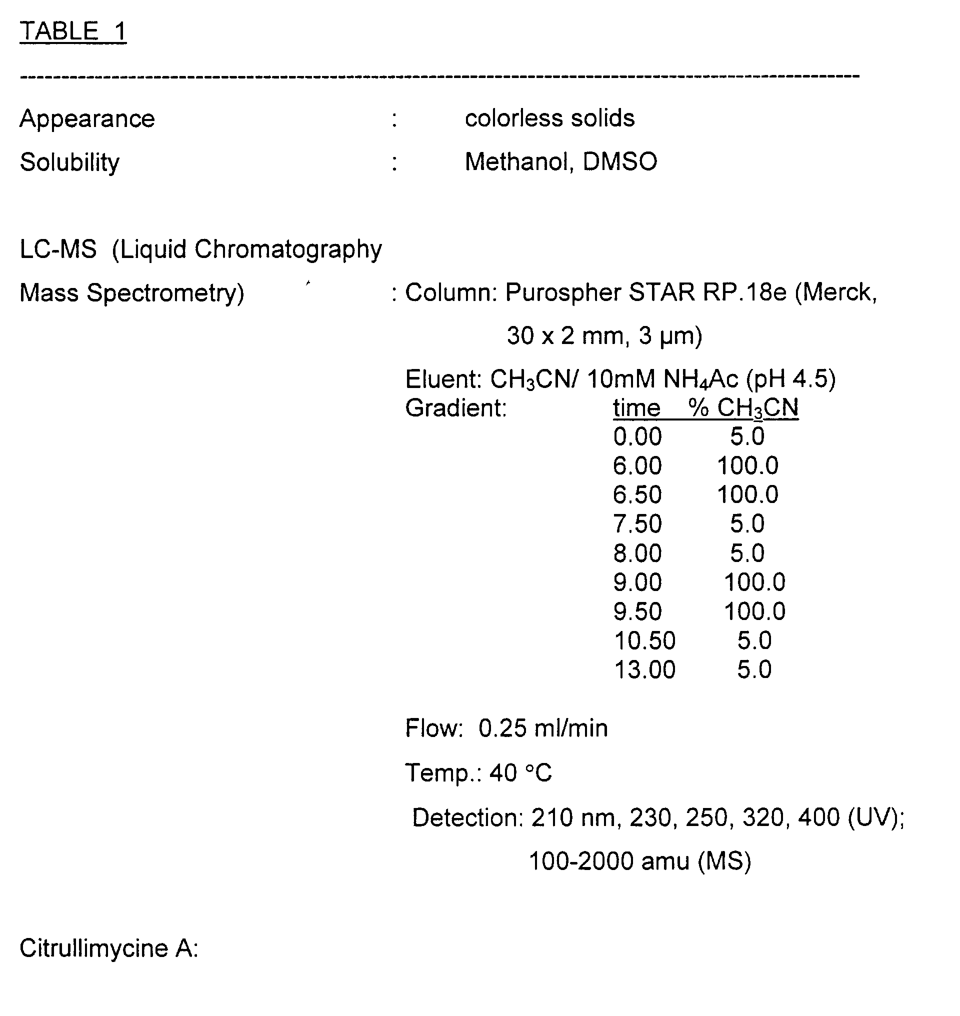

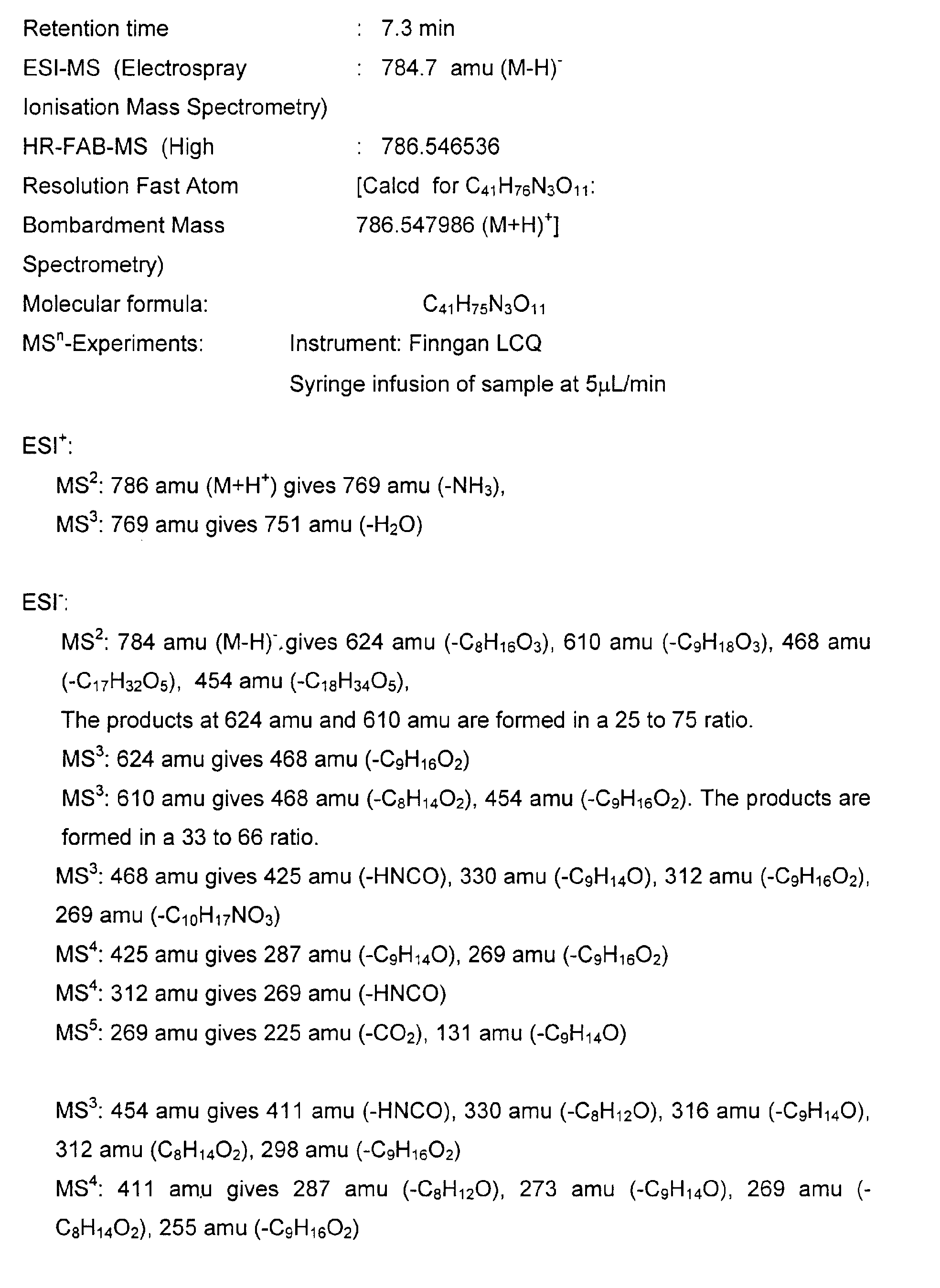

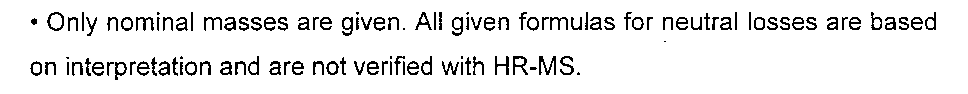

- ¹H NMR:: see Table 2
- ¹³C NMR:: see Table 2

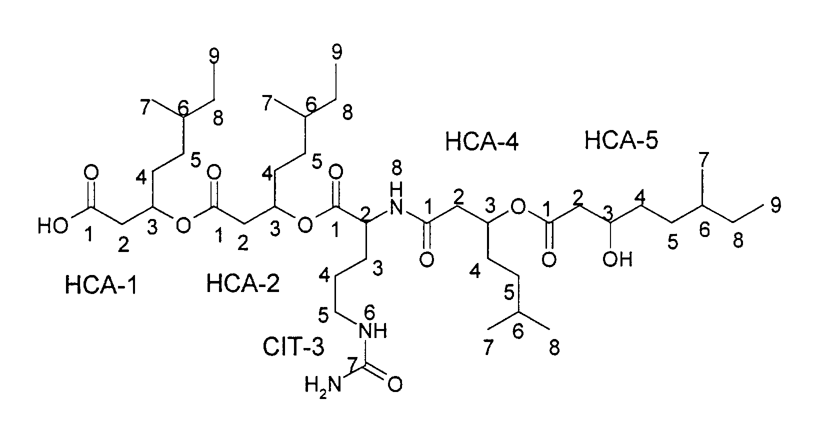

### Structure of isomer 1 of Citrullimycine A

| Citrullimycine B: | |
|---|---|
| Retention time | 7.1 min |
| Molecular formula | C₄₀H₇₃N₃O₁₁ |
| ESI-MS (Electrospray | 770 amu (M-H)⁻ |
| Ionisation Mass Spectrometry) | |

### MSⁿ Experiments

ESI⁻:
MS²: 770 amu (M-H)⁻ gives 610 amu (-C₈H₁₆O₃), 596 amu (-C₉H₁₈O₃), 468 amu, 454 amu, 440 amu.
The products at 610 amu and 596 amu are formed in a 1 : 1 ratio, the products at 468 amu, 454 amu and 440 amu are formed in a ratio of 15 : 58 : 27.
MS³: 610 amu gives 468 amu (-C₈H₁₄O₂), 454 amu (-C₉H₁₆O₂), 440 amu (-C₁₀H₁₈O₂). the products are formed in a ratio of 21 : 62 : 17.
MS³: 596 amu gives 468 amu (-C₇H₁₂O₂), 454 amu (-C₈H₁₄O₂), 440 amu (-C₉H₁₆O₂). Ratio: 6 : 51 : 43.
MS³: 468 amu gives 425 amu (-HNCO), 330 amu (-C₉H₁₄O), 312 amu (-C₉H₁₆O₂), 298 amu (-C₁₀H₁₈O₂)
MS³: 454 amu gives 411 amu (-HNCO), 330 amu (-C₈H₁₂O), 316 amu (-C₉H₁₄O), 312 amu (C₈H₁₄O₂), 298 amu (-C₉H₁₆O₂)
MS³: 440 amu gives 397 amu (-HNCO), 316 amu (-C₈H₁₂O), 298 amu (-C₈H₁₄O₂), 284 amu (-C₉H₁₆O₂)

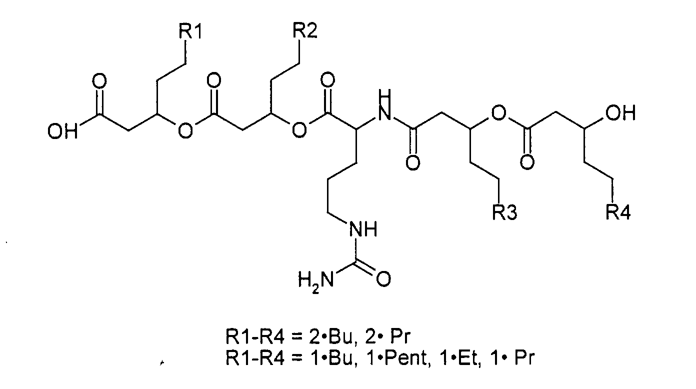

### Structure of Citrullimycin B (mixture of isomers with MW=771 amu)

| Citrullimycine C: | |
|---|---|
| Retention time | 7.1 min |
| Molecular formula | C₄₂H₇₇N₃O₁₁ |
| ESI-MS (Electrospray | 798 amu (M-H)⁻ |
| Ionisation Mass Spectrometry) | |

### MSⁿ Experiments

ESI⁻:
MS²: 798 amu (M-H)⁻ gives 638 amu (-C₈H₁₆O₃), 624 amu (-C₉H₁₈O₃), 610 amu (-C₁₀H₂₀O₃) The products are formed in the ratio of 5 : 90 : 5.
MS³: 624 amu gives 482 amu (-C₈H₁₄O₂), 468 amu (-C₉H₁₆O₂), 454 amu (-C₁₀H₁₈O₂), 440 amu (-C₁₁H₂₀O₂). Ratio: 1.6 : 96 : 2 : 0.4 .
MS⁴: 468 amu gives 425 amu (-HNCO), 330 amu (-C₉H₁₄O), 312 amu (-C₉H₁₆O₂), 287 amu (-C₁₀H₁₅O₂N), 269 amu (-C₁₀H₁₇O₃N)
MS⁵: 425 amu gives 287 amu (-C₉H₁₄O), 269 amu (-C₉H₁₆O₂)
MS⁵: 312 amu gives 269 amu (-HNCO)
MS⁵: 269 amu gives 225 amu (-CO₂), 131 amu (-C₉H₁₄O)

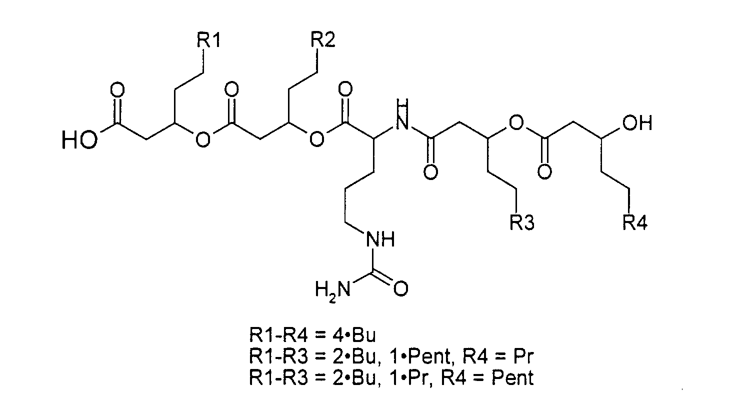

### Structure of Citrullimycin C (mixture of isomers, MW=799 amu)

**Table 2:**

| ¹H and ¹³C NMR Spectroscopic Data of Citrullimycine A in MeOD at 300 K^{a)}. | | |
|---|---|---|
| | ¹H | ¹³C |
| HCA1-1^{a)} | - | 171.58^{a)} |
| 2 | 2.59 | 40.30 |
| 3 | 5.18 | 72.21 |
| 4 | 1.62 | 32.90 |
| 5 | b) | b) |
| 6 | b) | b) |
| 7 | 0.87 | 19.45 |
| 8 | 1.35/1.15 | 30.51 |
| 9 | 0.87 | 11.73 |
| HCA2-1^{a)} | - | 171.44^{a)} |
| 2 | 2.58 | 40.24 |
| 3 | 5.16 | 72.61 |
| 4 | 1.62 | 32.90 |
| 5 | b) | b) |
| 6 | b) | b) |
| 7 | 0.87 | 19.45 |
| 8 | 1.35/1.15 | 30.51 |
| 9 | 0.87 | 11.73 |
| CIT3-1 | - | 175.81 |
| 2 | 4.33 | 53.99 |
| 3 | 1.87/1.68 | 30.25 |
| 4 | 1.55 | 27.73 |
| 5 | 3.12 | ∼40.6 (a) |
| 6 | - | - |
| 7 | - | 162.21 |
| HCA4-1 | - | 172.31 |
| 2 | 2.55/2.48 | 41.51 |
| 3 | 5.19 | 73.35 |
| 4 | 1.63 | 32.54 |
| 5 | 1.22 | 35.45 |
| 6 | 1.54 | 29.05 |
| 7 | 0.89 | 23.00 |
| 8 | 0.89 | 23.00 |
| HCA5-1 | - | 172.85 |
| 2 | 2.44 | 43.74 |
| 3 | 3.94 | 69.65 |
| 4 | 1.47 | 35.69 |
| 5 | 1.36 | 33.40 |
| 6 | b) | b) |
| 7 | 0.87 | 19.45 |
| 8 | 1.35/1.15 | 30.51 |
| 9 | 0.87 | 11.73 |

| | | |
|---|---|---|
| a) The data of HCA1 und HCA2 are interchangeable. | | |
| b) No assignment possible. | | |

### Example 4:

### Bioactivity Assay

### SPA [3H] Neurotensin Receptor binding Assay:

Compounds with an affinity for the neurotensin receptor will displace the binding of [3H] neurotensin which results in a diminished radioactive signal. For the SPA method, receptors which are immobilized directly to PVT WGA (Wheat Germ Agglutinin) coated SPA beads (Amersham pharmacia) should bind the radiolabelled ligand. The ligand-receptor complex will be held in close enough proximity to stimulate the beads to emit light. Any unbound radioligand is too distant from the bead to transfer energy and therefore will not be detected. The samples were prediluted 1:5 with assay buffer (50 mM Tris-HCI buffer with 1mM EDTA, 0.2 mM Bacitracin and 0.1 % BSA, pH 7.4) in deep well plates. The final dilution in the assay was 1:20.

96-well isoplates from Wallac were used for the screening. Each well received: 50 µl of sample, 50 µl of membrane in assay buffer (final conc. 16µg/well), 50 µl of PVT-VGA beads (final conc. 0.75 µg/well) and 50µl of 4 nM [³H]neurotensin. The plates were sealed and incubated for 2 hours on a shaker (1100 rpm) at room temperature. Prior to counting (MicroBeta Trilux, Wallac) the beads were allowed to settle for at least 20 minutes.

On each plate four wells without samples were used to determine the total receptor ligand binding and another four wells with 1 µM (L-α, γ-diaminobutyryl) neurotensin were used to determine the nonspecific binding respectively. Inhibition activities are expressed as:$\text{[1-(dpm sample―dpm nonspecific) / (dpm total binding-dpm nonspec.)] x 100 (%)}$

The activity of the Citrullimycines is in the range of 16- 30µM. The IC₅₀ of Citrullimycine A was determined as 16µM.

## Claims

1. A compound of the formula I: 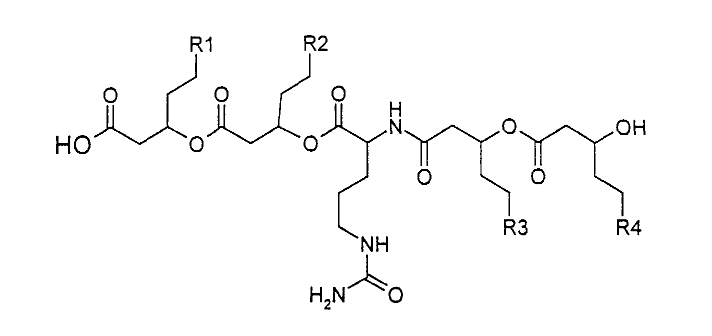 where
R₁, R₂, R₃ and R₄ are, independently of one another, alkyl residues with 1 to 6 carbon atoms; and the physiologically tolerated salts and derivatives thereof, in all their stereoisomeric forms and all their tautomeric forms.

2. A compound of the formula I as claimed in claim 1 or claim 2, in which one, two, three or all of R₁ to R₄ are butyl residues, and the physiologically tolerated salts and derivatives thereof, in all their stereoisomeric forms and all their tautomeric forms.

3. A compound of the formula I as claimed in any one of the preceding claims in which R₁ to R₄ consist of three butyl residues and one propyl residue, and the physiologically tolerated salts and derivatives thereof, in all their stereoisomeric forms and all their tautomeric forms.

4. A compound of the formula I as claimed in any one of claims 1 to 3, in which R₁ to R₄ comprise two butyl and two propyl residues or one butyl, one pentyl, one ethyl and one propyl residue, and the physiologically tolerated salts and derivatives thereof, in all their stereoisomeric forms and all their tautomeric forms.

5. A compound of the formula I as claimed in any one of claims 1 to 3, in which R₁ to R₄ comprise four butyl residues or two butyl, one propyl and one pentyl residue, and the physiologically tolerated salts and derivatives thereof, in all their stereoisomeric forms and all their tautomeric forms.

6. A compound of the formula I as claimed in any one of the preceding claims comprising two or more isomers.

7. A compound of the formula 1 or a physiologically tolerated salt or derivative thereof as claimed in one or more of claims 1 to 6, obtainable by cultivating Streptomycete sp., ST 101396 (DSM 13309) or one of its variants or mutants under aerobic conditions in a nutrient medium containing sources of carbon and nitrogen, isolating and purifying one or more target compounds in a customary manner, and converting where appropriate into physiologically tolerated salts or derivatives, in all their stereoisomeric and tautomeric forms.

8. A process for the production of a compound of the formula I or a salt or equivalent thereof as claimed in one or more of claims 1 to 6, comprising cultivating streptomycete sp., ST 101396 (DSM 13309) or one of its variants or mutants under aerobic conditions in a nutrient medium containing sources of carbon and nitrogen, isolating and purifying one or more target compounds in a customary manner, and converting where appropriate into physiologically tolerated salts or derivatives, in all their stereoisomeric and tautomeric forms.

9. The process as claimed in claim 8, wherein the cultivation is carried out at a temperature between 20 - 35°C and a pH between 5 and 8.

10. A compound of the formula I or a physiologically tolerated salt or derivative thereof as claimed in one or more of claim 1 to 6 for use as a pharmaceutical.

11. A compound of the formula I or a physiologically tolerated salt or derivative thereof as claimed in one or more of claim 1 to 6 for use as an inhibitor of neurotensin receptor.

12. The use of a compound of the formula I or a physiologically tolerated salt thereof as claimed in one or more of claims 1 to 6 for the manufacture of a pharmaceutical for the treatment of schizophrenia , Parkinson's or Alzheimer's disease.

13. A pharmaceutical composition, comprising an effective amount of a compound of the formula I as claimed in one or more of claims 1 to 6, or a pharmaceutically acceptable salt or derivative thereof, and a pharmaceutically acceptable carrier.

14. A method for the production of a pharmaceutical as claimed in claim 13, which comprises converting at least one compound of the formula I or a physiologically tolerated salt thereof as claimed in one or more of claims 1 to 6 with suitable excipients and/or carriers into a suitable dosage form.

15. Streptomycetes species ST 101396 (DSM 13309).
